# EUROPEAN PATENT SPECIFICATION

(11) **EP 0 783 342 B1**
(45) Date of publication and mention of the grant of the patent: **04.11.1998**
(21) Application number: 95934784.0
(22) Date of filing: 27.09.1995
(51) Int. Cl.: A61M 37/00

(54) **SAFETY INJECTION DEVICE**
SICHERE INJEKTIONSVORRICHTUNG
DISPOSITIF D'INJECTION A SECURITE

(30) Priority: 27.09.1994 US 312893
(43) Date of publication of application: 16.07.1997
(73) Proprietor: DELAB, 75016 Paris (FR)
(72) Inventor: CHERIF-CHEIKH, Roland, F-92130 Issy-les-Moulineaux (FR)
(74) Representative: Vossius, Volker, Dr.
(86) International application number: PCT/IB95/00961
(87) International publication number: WO 96/09849

(56) References cited:
- EP-A- 0 292 936
- EP-A- 0 415 504
- FR-A- 1 168 371
- NL-A- 8 901 124
- US-A- 3 016 895
- US-A- 4 359 044

## Description

### Field of the Invention

The present invention relates to injection devices and, in particular, to a device for the intramuscular or subcutaneous injection of a pharmaceutically active compound.

### Background of the Invention

The parenteral introduction of pharmaceutically active compounds is preferred over oral dosage in many applications, e.g. when the drug to be administered would partially or totally degrade in the gastrointestinal tract or where there is need for rapid response in emergency cases.

While parenteral administration is desirable in many applications, there are several drawbacks as it is currently practiced. One disadvantage is the discomfort felt by the patient to whom the drug is being administered. Parenteral preparations generally contain a large volume of liquid in which the drug is suspended or dissolved. When the active ingredient is poorly soluble or difficult to suspend in the carrier or when the active ingredient has to be administered at high doses, a fairly large volume of liquid must be injected. Ratios of active ingredient to carrier commonly run from 1:100 to 1:1000. Both the size of the needle and the volume of liquid being injected cause the resulting discomfort. Depending on its nature, the carrier itself may also be cause of pain.

A further disadvantage to administration of drugs in a liquid carrier is that the drugs are frequently not stable in the liquid. Therefore, the liquid and drug must be mixed substantially contemporaneously with injection. This can be of substantial disadvantage when, for example, many hundreds of people must be treated over a course of days in order to stem an epidemic (e.g. the administration of a vaccine).

Drugs in solid form rather than liquid form have been used for prolonged or controlled release formulations. The formulation can be an implant or a rod which is injected directly via a trocar, see for example European Patent Application Publication No. 0292936. However, trocars and the devices as set forth in this cited European Patent have some disadvantages. When the formulation is a prolonged or controlled formulation, the formulation must contain both the daily dose of drug multiplied by the number of days of activity of the drug and the amount of carrier necessary to control the rate of delivery of that drug for this period of time. Thus, this injected formulation requires a needle significantly larger than ordinary needles used with syringes, resulting in a painful injection.

NL-A-8 901 124 relates to an implanter for implanting an implant, e.g. a medicinal preparation or an electric circuit for remote detection, in an animal. An injection device is describes which comprises a hollow main body member, a hollow needle, a plunger and a hollow sleeve. NL-A-8 901 124 does not disclose a rod.

EP-A-0 415 504 describes an implanter which comprises a housing, a hollow injection needle which can be mounted on the housing, a plunger, spring means, and locking means comprising unlocking means. The locking means are for retaining the plunger against the pressure of spring means in a position where the plunger extends into the hollow needle. The unlocking means abut the body of the animate being when the injection needle has been introduced into the body, and subsequently automatically unlock the locking means for retaining the implant when the injection needle is being retracted.

### Summary of the Invention

The applicant has discovered a comparatively inexpensive device for the administration to a mammal, e.g., a human, of solid or substantially solid drugs by the parenteral route wherein the drugs are intended to be immediately assimilated by the body.

Because the quantity of drug can just be merely the amount needed for an immediate effect and because there may be no need for carriers, the needle can be as small as ordinary needles. The drug is already stable in a solid form and, thus, no contemporaneous mixing is needed. The injection is less painful because the injected volume is dramatically reduced as compared to the volume needed for liquid injection. The risk of contamination is also reduced because there is no premanipulation of a liquid injectable formulation before making the injection. A seal can be fitted on the tip of the injection member to insure complete sterility until injection through the skin.

The device of the present invention has a needle which is not exposed to the outside elements. Thus, the needle will not pick up airborne contaminants, nor inadvertently scratch someone, such as a hospital worker, and either inadvertently disburse the medicament or the blood of the patient to the hospital worker. This aspect is especially important for treatment of unstable patients.

Other features and advantages of the invention will be apparent from the drawings, the detailed description, and from the claims.

### Brief Description of the Drawings

Fig. 1 shows the device at rest;
Fig. 2 shows the device with the needle injected into the patient;
Fig. 3 shows the needle being withdrawn with the medicament remaining in the patient;
Fig. 4 shows complete withdrawal of the needle from the patient; and
Fig. 5 shows a cross-section through line 5-5 of Fig. 1.

### Detailed Description

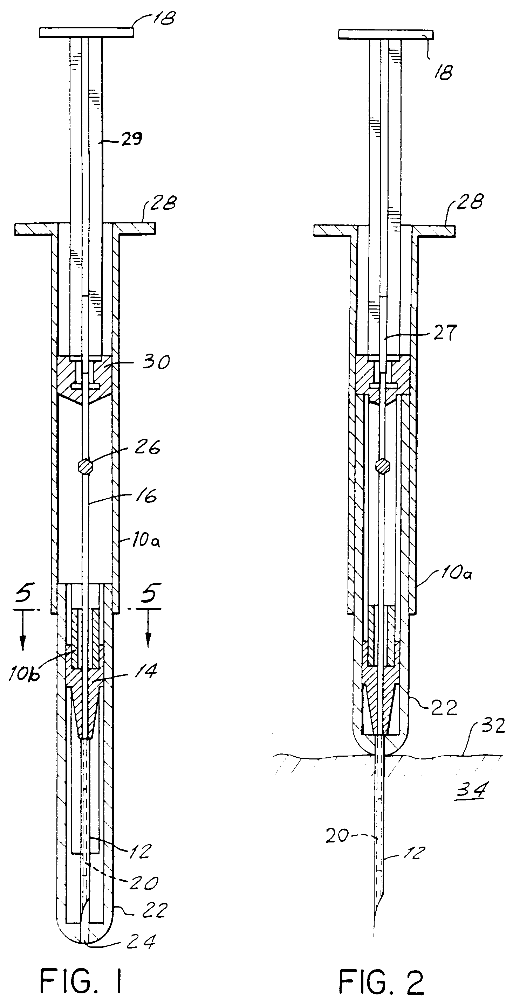

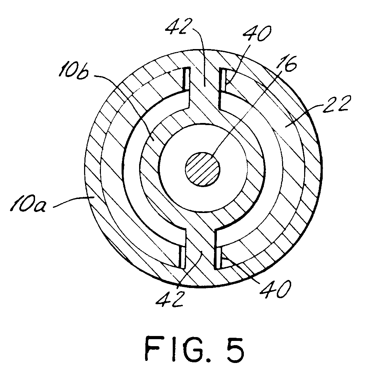

Referring first to Fig. 1, there is shown an injection device 1 including main body member 10a, 10b which is attached to needle 12 through coupling means 14. A rod 16 is guided into needle 12 and abuts medicament 20 which is positioned in needle 12. A sleeve 22 surrounds needle 12 so that needle 12 is not exposed until used. Sleeve 22 is provided with one or more slots 40 (see Fig. 5) along its length so that main body members 10a and 10b can be joined by radially extending connecting members 42. A seal 24 can cover the opening 23 and the slots 40 of sleeve 22 in order to maintain the sterility of needle 12 and medicament 20. The seal is preferably of easily friable material such as wax, but can also be a cap over the entire sleeve 22. Rod 16 includes a bulge 26 which serves as a stopper for travel of the rod 16. Main body member 10a has a flange 28 to assist in removal of the device after injection. Rod 16 is attached to a plunger 29 which has a top thumb flange 18 and a bottom longitudinal bore 27. A guide 30 is attached to plunger 29 and is positioned in main body member 10a to guide plunger 29 and rod 16.

Fig. 2 shows the device of Fig. 1 wherein needle 12 has penetrated the skin 32 of the person being treated. As shown, needle 12 has penetrated through the skin 32 into the subcutaneous layer 34. As the device of Fig. 1 is pressed against the skin 32, sleeve 22 is retracted into main body member 10a by the force of pressure against the skin 32.

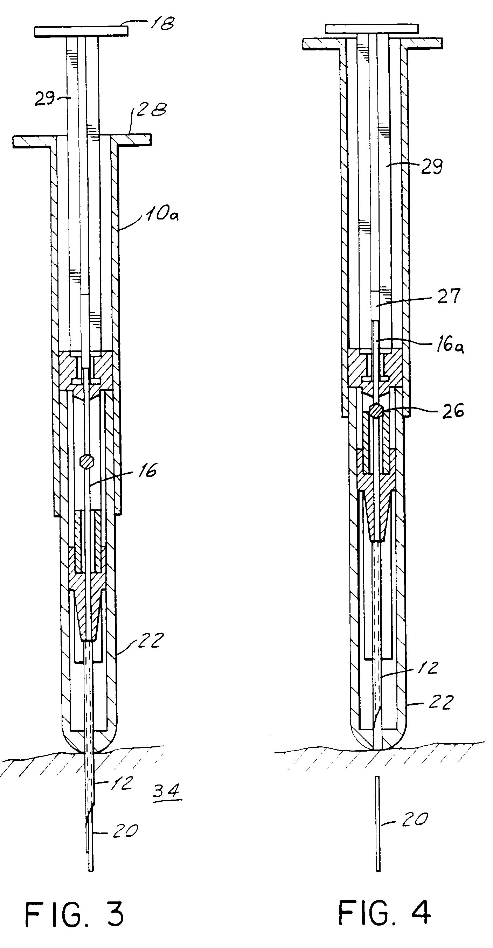

At this point, and as shown in Fig. 3, main body member 10a is moved in an upward direction by exerting finger pressure against the lower part of flange 28 while simultaneously exerting opposing pressure with the thumb on flange 18 of plunger 29. This relative movement of the plunger 29 and the main body member 10a causes the needle 12 to retract into sleeve 22 thus leaving medicament 20 in the subcutaneous layer 34 of the patient.

Fig. 4 shows the needle 12 fully withdrawn into sleeve 22 and with medicament 20 remaining in the subcutaneous layer 34 of the patient. As can also be seen in Fig. 4, the upper portion 16a of rod 16 has been pushed into a bore 27 in plunger 29 by the action of coupling means 14 against bulge 26 of rod 16.

Fig. 5 is a cross-sectional view of Fig. 1 at 5-5. Fig. 5 shows slots 40 in sleeve 22. Radially extending connecting members 42 extend through the slots 40 to join main body member 10a with main body member 10b.

Medicament 20 is solid or semi-solid. The amount of carrier, if present, should be as small as possible. The amount of active ingredient in the medicament 20 is generally at least 20% and is preferably above 50%. With suitable medicaments which will maintain a shape, the amount of active ingredient can be up to 100%.

The active ingredient can be a peptide or a protein. Examples of such peptides include growth hormone releasing peptide (GHRP), luteinizing hormone-releasing hormone (LHRH), somatostatin, bombesin, gastrin releasing peptide (GRP), calcitonin, bradykinin, galanin, melanocyte stimulating hormone (MSH), growth hormone releasing factor (GRF), amylin, tachykinins, secretin, parathyroid hormone (PTH), enkephalin, endothelin, calcitonin gene releasing peptide (CGRP), neuromedins, parathyroid hormone related protein (PTHrP), glucagon, neurotensin, adrenocorticotrophic hormone (ACTH), peptide YY (PYY), glucagon releasing peptide (GLP), factor VIII, vasoactive intestinal peptide (VIP), pituitary adenylated cyclase activating peptide (PACAP), motilin, substance P, neuropeptide Y (NPY), TSH, and analogs and fragments thereof. Other active ingredients include insulin, adrenaline, xylocaine, morphine, a glucocorticoid (e.g., dexamethasone), atropine, a cystostatic compound, estrogen, androgen, interleukins, digitoxin, biotin, testosterone, heparin, cyclosporin, penicillin, vitamins, anti-platelet activating factor agents (e.g., ginkgolides), or diazepam.

In order to provide instant delivery of the active ingredient, the carrier should be water soluble. Examples of water soluble carriers include hyaluronic acid, cellulose, (e.g., hydroxypropylmethyl cellulose (HPMC), carboxymethyl cellulose (CMC), and hydroxyethyl cellulose (HEC)), polyalcohols (e.g., mannitol), sugars (e.g., dextrose, mannose, and glucose), gelatin, polyvinylpyrrolidone, and starches. The carrier may also be water insoluble, but biodegradable to provide sustained delivery. Examples of suitable carriers include water insoluble polyesters of L-lactic acid, D-lactic acid, DL-lactic acid, L-lactide, D-lactide, DL-lactide, glycolide, glycolic acid, capralactone, and any optically active isomers, racemates, or copolymers thereof.

The medicament may be prepared by conventional techniques such as compression, thermofusion, or extrusion. Compression suitably consists of a tabletting process in which a rod-shaped medicament 20 is formed. Thermofusion suitably consisting of mixing and melting of the active ingredient and a carrier, if desired. The melted product is then molded into the rod-shaped medicament. Extrusion suitably consists of mixing the active ingredients and carrier, if desired, with a liquid to form a semisolid paste. The paste is then forced through a small diameter opening to form a rod and then cut at its desired length. The diameter of the medicament 20 may be up to 2 mm but is preferably from about 0.25 to about 0.5 mm in diameter and about 1 to about 3 cm in length. The diameter of the rod 16 is preferably about the same diameter as the diameter of the medicament 20. The inside diameter of the needle 12 is preferably just slightly larger than the diameter of the medicament. It is preferred that the needle 12 and rod 16 be metallic, notably stainless steel; the balance of the components of the device can be relatively inexpensive plastic materials.

A lid or cover can serve as the seal when placed around the sleeve 22 to both prevent accidental movement of sleeve 22 and insure sterility of the medicament 20 and needle 12. The injection device may be stored in a conventional blister pack.

### Other Embodiments

It will be understood that while the invention has been described in conjunction with the detailed description thereof, that the foregoing description is intended to illustrate and not limit the scope of the invention, which is defined by the scope of the appended claims.

## Claims

1. An injection device for injecting a solid or semi-solid medicament parenterally into a mammal, said device comprising
a hollow main body member (10a, 10b),
a hollow needle (12) affixed to a distal end of said main body member (10a, 10b) and arranged to hold the medicament,
a rod (16) slidably arranged within said needle (12), said rod (16) extending longitudinally within said main body member (10a, 10b), wherein said rod (16) includes a bulge (26) which functions to limit travel of said rod (16) within said main body member (10a, 10b),
a plunger (29) affixed to a proximal end of said rod (12) and arranged to slide within a proximal end of said main body member (10a, 10b), and
a hollow sleeve (22) slidably positioned at said distal end of said main body member (10a, 10b) to enclose said needle (12) when in an extended position,
wherein when said device is pressed against skin of the mammal said sleeve (22) slides along said main body member (10a, 10b) thereby exposing said needle (12) and allowing said needle to penetrate the skin, and wherein said plunger (29) and rod (16) are slidably arranged to maintain the medicament in the mammal as the needle (16) is withdrawn from the mammal.

2. The device of claim 1, wherein said plunger (29) includes a flange.

3. The device of claim 1, wherein said sleeve (22) slides within said main body member.

4. The device of claim 1, wherein said main body member (10a, 10b) includes a flange (28).

5. The device of claim 1, wherein said sleeve (22) includes a seal (24) operative to maintain the sterility of the needle.

6. The device of claim 5, wherein said seal (24) comprises a cap that covers said sleeve (22).

7. The device of claim 1, wherein said needle contains a medicament (20).

8. The device of claim 7, wherein said medicament contains a peptide.

9. The device of claim 1, wherein said device is enclosed in a blister pack.

## Patentansprüche

1. Eine Injektions-Vorrichtung zur parenteralen Injektion eines festen oder halbfesten Arzneimittels in einen Säuger, wobei die Vorrichtung umfaßt:
ein hohles Hauptkörperelement (10a, 10b),
eine hohle Nadel (12), die an einem distalen Ende des Hauptkörperelements (10a, 10b) befestigt und dazu eingerichtet ist, das Arzneimittel zu enthalten,
einen gleitbar eingerichteten Stab (16) innerhalb der Nadel (12), wobei der Stab sich in Längsrichtung innerhalb des Hauptkörperelements (10a, 10b) erstreckt, wobei der Stab (16) eine Wulst (26) enthält, die zur Begrenzung des Hubs des Stabs (16) innerhalb des Hauptkörperelements (10a, 10b) fungiert,
einen Kolben (29), der an einem proximalen Ende des Stabs (16) befestigt und dazu eingerichtet ist, innerhalb eines proximalen Endes des Hauptkörperelements (10a, 10b) zu gleiten, und
eine hohle Buchse (22), die gleitbar am distalen Ende des Hauptkörpers (10a, 10b) angeordnet ist, um die Nadel (12) einzuschließen, wenn sie sich in einer ausgestreckten Lage befindet, wobei die Buchse (22), wenn die Vorrichtung gegen die Haut des Säugers gedrückt wird, entlang des Hauptkörperelements (10a, 10b) gleitet, wodurch die Nadel (12) freigelegt und ihr erlaubt wird, in die Haut einzudringen, und wobei der Kolben (29) und der Stab (16) gleitbar eingerichtet sind, um das Arzneimittel in dem Säuger zu belassen, wenn die Nadel (12) aus dem Säuger zurückgezogen wird.

2. Die Vorrichtung nach Anspruch 1, wobei der Kolben (29) einen Flansch enthält.

3. Die Vorrichtung nach Anspruch 1, wobei die Buchse (22) innerhalb des Hauptkörperelements gleitet.

4. Die Vorrichtung nach Anspruch 1, wobei das Hauptkörperelement (10a, 10b) einen Flansch enthält.

5. Die Vorrichtung nach Anspruch 1, wobei die Buchse (22) eine Abdichtung (24) enthält, die die Aufgabe hat, die Sterilität der Nadel aufrechtzuerhalten.

6. Die Vorrichtung nach Anspruch 5, wobei die Abdichtung (24) eine Kappe umfaßt, die die Buchse (22) bedeckt.

7. Die Vorrichtung nach Anspruch 1, wobei die Nadel ein Arzneimittel (20) beinhaltet.

8. Die Vorrichtung nach Anspruch 7, wobei das Arzeimittel ein Peptid beinhaltet.

9. Die Vorrichtung nach Anspruch 1, wobei die Vorrichtung in einer Blasenverpackung eingeschlossen ist.

## Revendications

1. Dispositif d'injection pour l'injection d'un médicament solide ou semi-solide par voie parentérale chez un mammifère, ledit dispositif comprenant
un corps principal creux (10a, 10b),
une aiguille creuse (12) fixée à une extrémité distale du dit corps principal (10a, 10b) et agencée pour véhiculer le médicament,
une tige (16) agencée de façon à coulisser dans ladite aiguille (12) , ladite tige (16) s'étendant longitudinalement dans ledit corps principal (10a, 10b), tandis que ladite tige (16) comporte un renflement (26) qui permet de limiter le parcours de ladite tige (16) dans ledit corps principal (10a, 10b),
un piston (29) fixé à l'extrémité proximale de ladite tige (12) et agencé pour coulisser dans une extrémité proximale du dit corps principal (10a, 10b), et
une gaine creuse (22) placée de façon à coulisser à ladite extrémité distale du dit corps principal (10a, 10b) pour enfermer ladite aiguille (12) lorsqu'elle est en position sortie,
dans lequel, lorsque ledit dispositif est pressé contre la peau du mammifère, ladite gaine (22) coulisse le long du dit corps principal (10a, 10b), exposant ainsi ladite aiguille (12) et permettant à ladite aiguille de pénétrer dans la peau, et dans lequel lesdits piston (29) et tige (16) sont agencés en coulissement de façon à maintenir le médicament dans le mammifère lorsque l'aiguille (16) est retirée du mammifère.

2. Dispositif selon la revendication 1, dans lequel ledit piston (29) comporte une bride.

3. Dispositif selon la revendication 1, dans lequel ladite gaine (22) coulisse dans ledit corps principal.

4. Dispositif selon la revendication 1, dans lequel ledit corps principal (10a, 10b) comprend une collerette (28).

5. Dispositif selon la revendication 1, dans lequel ladite gaine (22) comprend une obturation (24) destinée à maintenir la stérilité de l'aiguille.

6. Dispositif selon la revendication 5, dans lequel ladite obturation (24) comprend un opercule qui recouvre ladite gaine (22).

7. Dispositif selon la revendication 1, dans lequel ladite aiguille contient un médicament (20).

8. Dispositif selon la revendication 7, dans lequel ledit médicament contient un peptide.

9. Dispositif selon la revendication 1, dans lequel ledit dispositif est enfermé dans un emballage sous blister.
